# EUROPÄISCHE PATENTSCHRIFT

(11) **EP 3 329 895 B1**
(45) Veröffentlichungstag und Bekanntmachung des Hinweises auf die Patenterteilung: **15.09.2021**
(21) Anmeldenummer: 17204588.2
(22) Anmeldetag: 30.11.2017
(51) Int. Cl.: A61H 7/00, A61H 11/00, A61F 7/00

(54) **PORTABLES MASSAGEGERÄT**
PORTABLE MASSAGE APPARATUS
APPAREIL DE MASSAGE PORTABLE

(30) Priorität: 30.11.2016 DE 202016106680 U
(43) Veröffentlichungstag der Anmeldung: 06.06.2018
(73) Patentinhaber: Grbic, Margita, 80995 München (DE)
(72) Erfinder: Grbic, Margita, 80995 München (DE)
(74) Vertreter: Kudla, Christof

(56) Entgegenhaltungen:
- DE-A1- 2 607 430
- JP-A- 2002 065 791
- JP-A- 2006 320 630
- KR-B1- 101 440 088
- US-A- 5 063 911
- US-A- 5 305 738

## Beschreibung

Die Erfindung betrifft ein portables Massagegerät mit einem formveränderlichen Kontaktsegment zur Auflage auf einen zu massierenden Körperbereich und einem oder mehreren elektrisch antreibbaren Massageorganen, welche innerhalb eines Grundkörpers angeordnet und dazu eingerichtet sind, im Betrieb eine Formveränderung des Kontaktsegments zu verursachen.

Die Erfindung betrifft außerdem ein Kontaktsegment für ein portables Massagegerät.

Portable Massagegeräte werden beispielsweise zur Massage des Nackens, der Schultern, der Schulterblätter, des Rücken, der Hüfte, der Beine, des Bauchs oder der Füße eingesetzt und dienen der Entspannung und Steigerung des Wohlbefindens.

Das Dokument KR101440088B1 offenbart ein portables Massagegerät, das alle in dem Oberbegriff des Anspruchs 1 dargelegten technischen Merkmale aufweist.

Anders als stationäre Massagegeräte, wie beispielsweise fest installierte Massagebänke oder Massagestühle, können portable Massagegeräte an unterschiedlichen Orten oder sogar unterwegs verwendet werden.

Gattungsgemäße Massagegeräte weisen ein Kontaktsegment zur Auflage auf einen zu massierenden Körperbereich auf, wobei die Form des Kontaktsegments während des Betriebs eines oder mehrerer Massageorgane des Massagegeräts verändert wird. Durch die Auflage auf den zu massierenden Körperbereich und die andauernde Formveränderung des Kontaktsegments wird das Kontaktsegment aufgrund der entstehenden Reibung kontinuierlich mechanisch beansprucht.

Die kontinuierliche Beanspruchung führt zu einer gesteigerten Abnutzung des Kontaktsegments, wodurch die Optik des Kontaktsegments und die Massagewirkung des Massagegeräts auf Dauer beeinträchtigt werden.

Die der Erfindung zugrunde liegende Aufgabe besteht somit darin, ein Massagegerät bereitzustellen, dessen Wirkung und Optik auch bei einer intensiven Benutzung über einen langen Zeitraum aufrechterhalten werden kann.

Die Aufgabe wird gelöst mit einem Massagegerät der eingangs genannten Art, wobei das Kontaktsegment zerstörungsfrei und reversibel lösbar mit dem Grundkörper verbunden ist.

Die Erfindung macht sich die Erkenntnis zunutze, dass durch die zerstörungsfrei und reversibel lösbare Verbindung zwischen dem Grundkörper und dem Kontaktsegment die Möglichkeit geschaffen wird, das Kontaktsegment austauschen zu können, nachdem es aufgrund intensiver Benutzung eine Abnutzungsschwelle überschritten hat. Eine Notwendigkeit die Bestandteile des Massagegeräts, auf welche ein Großteil der Kosten des Massagegeräts entfallen, insbesondere das eine oder die mehreren elektrisch antreibbaren Massageorgane, besteht somit nicht, sodass die Kosten für die Aufrechterhaltung der Massagewirkung und der Optik des Massagegeräts durch den Austausch des Kontaktsegments ohne einen hohen finanziellen Aufwand möglich ist. Darüber hinaus vereinfacht die zerstörungsfrei und reversibel lösbare Verbindung zwischen dem Grundkörper und dem Kontaktsegment die Reinigung des Kontaktsegments.

In dem Massagegerät gemäß der Erfindung ist das Kontaktsegment mittels eines oder mehrerer Reißverschlüsse mit dem Grundkörper verbunden. Reißverschlüsse stellen eine mechanisch belastbare und gleichzeitig flexible zerstörungsfrei und reversibel lösbare Verbindung bereit, welche sich aufgrund der einfachen und schnellen Betätigbarkeit besonders für austauschbare Segmente eignen. Ferner können Reißverschlüsse kostengünstig hergestellt und/oder zugekauft werden und sind in unterschiedlichen Ausführungen und großen Stückzahlen verfügbar. Die jeweiligen Schiebergriffe der Reißverschlussschlitten sind vorzugsweise derart angeordnet, dass diese von dem Kontaktsegment oder dem Grundkörper verdeckt werden, sodass der Anwendungskomfort des Massagegeräts nicht beeinträchtigt wird.

Alternativ ist der eine oder sind die mehreren Reißverschlüsse schiebergrifflos ausgebildet.

Das erfindungsgemäße Massagegerät wird dadurch vorteilhaft weitergebildet, dass eine oder mehrere Krampenreihen entlang der Seitenkanten des Kontaktsegments verlaufen. Dadurch, dass eine oder mehrere Krampenreihen entlang der Seitenkanten des Kontaktsegments verlaufen, wird vermieden, dass der eine oder die mehreren Reißverschlüsse in dem Kontaktbereich des Kontaktsegments angeordnet sind, welcher in Berührung mit dem zu massierenden Körperbereich kommt, sodass eine Beeinträchtigung des Benutzungskomforts des Massagegeräts durch den einen oder die mehreren Reißverschlüsse vermieden wird. Außerdem führt die Anordnung einer oder mehrerer Krampenreihen entlang der Seitenkanten des Kontaktsegments zu einer mechanisch robusten Verbindung zwischen dem Grundkörper und dem Kontaktsegment, sodass die Lebensdauer des Massagegeräts gesteigert wird.

In einer bevorzugten Ausführungsform des erfindungsgemäßen Massagegeräts verläuft eine erste Krampenreihe entlang einer ersten Seitenkante des Kontaktsegments und eine zweite Krampenreihe verläuft entlang einer zweiten Seitenkante des Kontaktsegments, wobei die erste Seitenkante des Kontaktsegments im Wesentlichen gegenüberliegend zu der zweiten Seitenkante des Kontaktsegments angeordnet ist. Vorzugsweise weisen der Reißverschluss der ersten Krampenreihe und der Reißverschluss der zweiten Krampenreihe entgegengesetzte Öffnungsrichtungen auf. Somit wird vermieden, dass durch ein Ziehen an dem Kontaktsegment eine Öffnung der entsprechenden Reißverschlüsse erfolgen kann, sodass das Kontaktsegment sich von dem Grundkörper löst. Auf diese Weise wird das Risiko eines unbeabsichtigten Lösens des Kontaktsegments von dem Grundkörper erheblich verringert.

In einer weiteren Ausführungsform des erfindungsgemäßen Massagegeräts ist das Kontaktsegment mittels eines oder mehrerer Klettverschlüsse mit dem Grundkörper verbunden. Das Kontaktsegment kann zusätzlich zu dem einen oder den mehreren Klettverschlüssen ebenfalls mit einem oder mehreren Reißverschlüssen mit dem Grundkörper verbunden sein. Klettverschlüsse lassen sich besonders schnell öffnen und sind darüber hinaus äußerst kostengünstig. Das Lösen des Kontaktsegments von dem Grundkörper und somit auch der Austausch eines abgenutzten Kontaktsegments durch ein neues Kontaktsegment werden somit beschleunigt, wodurch der Anwendungskomfort weiter gesteigert wird.

Ferner ist ein erfindungsgemäßes Massagegerät bevorzugt, bei welchem das Kontaktsegment mittels eines oder mehrerer Druckknöpfe mit dem Grundkörper verbunden ist. Das Kontaktsegment kann zusätzlich zu dem einen oder den mehreren Druckknöpfen ebenfalls mit einem oder mehreren Reißverschlüssen und/oder mit einem oder mehreren Klettverschlüssen mit dem Grundkörper verbunden sein. Druckknöpfe sind besonders belastbar und weisen eine lange Lebensdauer auf. Insbesondere bei Kontaktsegmenten aus haltbaren und widerstandfähigen Materialien und einer daraus resultierenden vergleichsweise langen Lebensdauer ist die Verwendung von Druckknöpfen vorteilhaft.

In einer anderen Ausführungsform des erfindungsgemäßen Massagegeräts weist das Kontaktsegment einen Kontaktbereich auf, welcher dazu eingerichtet ist, den zu massierenden Körperbereich zu berühren. Der Kontaktbereich des Kontaktsegments ist zumindest teilweise aus einem Textilstoff ausgebildet. Vorzugsweise ist der Textilstoff maschinenwaschbar, sodass die Reinigung des Kontaktsegments vereinfacht wird. Der Textilstoff kann Naturfasern und/oder Chemiefasern aufweisen. Ferner kann der Textilstoff abschnittsweise oder vollständig als Gewebe, Gewirke, Gestricke, Geflecht oder als Vliesstoff oder Filz ausgebildet sein. Insbesondere weist der Textilstoff eine geringe Oberflächenrauigkeit auf, sodass bei dem Benutzer ein angenehmes Berührungsempfinden hervorgerufen und die durch die Formveränderung des Kontaktsegments hervorgerufene Reibwirkung minimiert wird.

In einer besonders bevorzugten Ausführungsform des erfindungsgemäßen Massagegeräts ist der Textilstoff als Netzstoff ausgebildet. Der Netzstoff ist vorzugsweise luft- und/oder feuchtigkeitsdurchlässig. Durch die Luftdurchlässigkeit wird eine ausreichende Belüftung des zu massierenden Körperbereichs gewährleistet, sodass der Massagekomfort erhöht wird. Durch die Feuchtigkeitsdurchlässigkeit kann zusätzlich ein Abtransport von Feuchtigkeit, wie beispielsweise Körperschweiß, von dem zu massierenden Körperbereich umgesetzt werden, wodurch das Massagegerät auch über einen längeren Zeitraum und bei höheren Temperaturen nutzbar ist.

Das erfindungsgemäße Massagegerät wird außerdem dadurch vorteilhaft weitergebildet, dass das Kontaktsegment zumindest abschnittsweise gepolstert ist. Die Polsterung ist dabei derart ausgebildet, dass das eine oder die mehreren Massageorgane im Betrieb weiterhin eine Formveränderung des Kontaktsegments verursachen, welche von der zu massierenden Person wahrnehmbar ist. Durch die Polsterung wird die Wirkung der Bewegung des einen oder der mehreren Massageorgane gedämpft, wodurch der Massagekomfort gesteigert wird. Außerdem kann auf elastisch verformbare beziehungsweise nachgiebige Massageaufsätze verzichtet werden, sodass die Herstellungskosten des Massagegeräts gesenkt werden.

In einer anderen Ausführungsform des erfindungsgemäßen Massagegeräts weist der Grundkörper zwei längliche Seitenteile auf, welche sich jeweils seitlich von dem Kontaktsegment erstrecken. Die zwei länglichen Seitenteile dienen beispielsweise der Stabilisierung des Massagegeräts während der Massage. Zum Halten des Massageräts in der gewünschten Position können die zwei länglichen Seitenteile beispielsweise von dem Benutzer gegriffen werden. Beim Verwenden des Massagegeräts zur Durchführung einer Nackenmassage können die zwei länglichen Seitenteile jeweils über eine Schulter des Benutzers gelegt werden, sodass die Enden der zwei länglichen Seitenteile etwa im Brustbereich des Benutzers gegriffen werden können. Alternativ kann eines der zwei länglichen Seitenteile oder können beide länglichen Seitenteile auch im unteren Oberkörperbereich seitlich an dem Körper vorbeilaufen, sodass die Enden der zwei länglichen Seitenteile etwa im Bauch- oder Hüftbereich des Benutzers gegriffen werden können. Auf diese Weise lässt sich das Massagegerät bei der Durchführung von unterschiedlichen Rückenmassagen angenehm vom Benutzer halten.

Bevorzugt ist außerdem ein erfindungsgemäßes Massagegerät, bei welchem die länglichen Seitenteile des Grundkörpers jeweils eine Schlaufe aufweisen. Die Schlaufen lassen sich besonders komfortabel und angenehm von dem Benutzer greifen und ermöglichen somit das Halten des Massagegeräts über einen längeren Zeitraum. Außerdem kann der Anpressdruck des Kontaktsegments auf den zu massierenden Körperbereich durch eine Zugkraftbeaufschlagung der Schlaufen durch den Benutzer auf besonders einfache Weise variiert werden, wodurch letztendlich die Massageintensität angepasst werden kann.

In einer weiteren Ausführungsform des erfindungsgemäßen Massagegeräts umfasst das eine oder umfassen die mehreren elektrisch antreibbaren Massageorgane einen oder mehrere Massageköpfe. Der eine oder die mehreren Massageköpfe können beispielsweise dazu eingerichtet sein, rotatorische und/oder lineare Bewegungen auszuführen. Insbesondere ist das eine oder sind die mehreren elektrisch antreibbaren Massageorgane mit einer Steuerungseinrichtung verbunden, mittels welcher der Antrieb des einen oder der mehreren Massageorgane steuerbar ist. Vorzugsweise ist die Steuerungseinrichtung dazu eingerichtet, die Bewegungsgeschwindigkeit und/oder die Bewegungsrichtung des einen oder der mehreren Massageorgane zu steuern.

Das erfindungsgemäße Massagegerät wird außerdem dadurch vorteilhaft weitergebildet, dass das eine oder die mehreren elektrisch antreibbaren Massageorgane dazu eingerichtet sind, eine Knetmassage auszuführen. Bei einer Knetmassage bewegt sich das eine oder bewegen sich die mehreren elektrisch antreibbaren Massageorgane in alle drei Raumrichtungen. Durch die Knetmassage kommt es zu einer Kräftigung der Muskulatur. Gleichzeitig kann die Durchblutung und/oder der Stoffwechsel angeregt werden. Die Knetmassage ist außerdem besonders bevorzugt beim Lösen von Rückenverspannungen und der Vorbeugung von Rückenproblemen.

In einer vorteilhaften Weiterbildung umfasst das erfindungsgemäße Massagegerät ein oder mehrere Heizelemente, welche innerhalb des Grundkörpers angeordnet und dazu eingerichtet sind, im Betrieb eine Erwärmung des Kontaktsegments zu verursachen. Durch die Erwärmung des Kontaktsegments wird der zu massierende Körperbereich erwärmt, sodass der Massagekomfort weiter gesteigert wird. Außerdem kann das Massagegerät durch das eine oder die mehreren Heizelemente zusätzlich zur Wärmetherapie eingesetzt werden. Vorzugsweise ist das eine oder sind die mehreren Heizelemente mit der Steuerungseinrichtung verbunden, wobei die Steuerungseinrichtung dazu eingerichtet ist, die Temperatur des einen oder der mehreren Heizelemente zu steuern.

Die der Erfindung zugrundeliegende Aufgabe wird ferner durch ein Kontaktsegment für ein portables Massagegerät gemäß einer der vorstehend beschriebenen Ausführungsformen gelöst. Das erfindungsgemäße Kontaktsegment dient beispielsweise dem Austausch von abgenutzten Kontaktsegmenten. Durch das Austauschen des Kontaktsegments eines Massagegeräts kann die Wirkung und die Optik auch bei einer intensiven Benutzung über einen langen Zeitraum aufrechterhalten werden.

Nachfolgend werden bevorzugte Ausführungsformen der Erfindung unter Bezugnahme auf die beiliegenden Zeichnungen näher erläutert und beschrieben. Dabei zeigen:
Fig. 1 ein Ausführungsbeispiel des erfindungsgemäßen Massagegeräts in einer schematischen Darstellung;
Fig. 2 das erfindungsgemäße Massagegerät aus Fig. 1 in einer weiteren schematischen Darstellung; und
Fig. 3 das Kontaktsegment des erfindungsgemäßen Massagegeräts aus Fig. 1 und Fig. 2 in einer schematischen Darstellung.

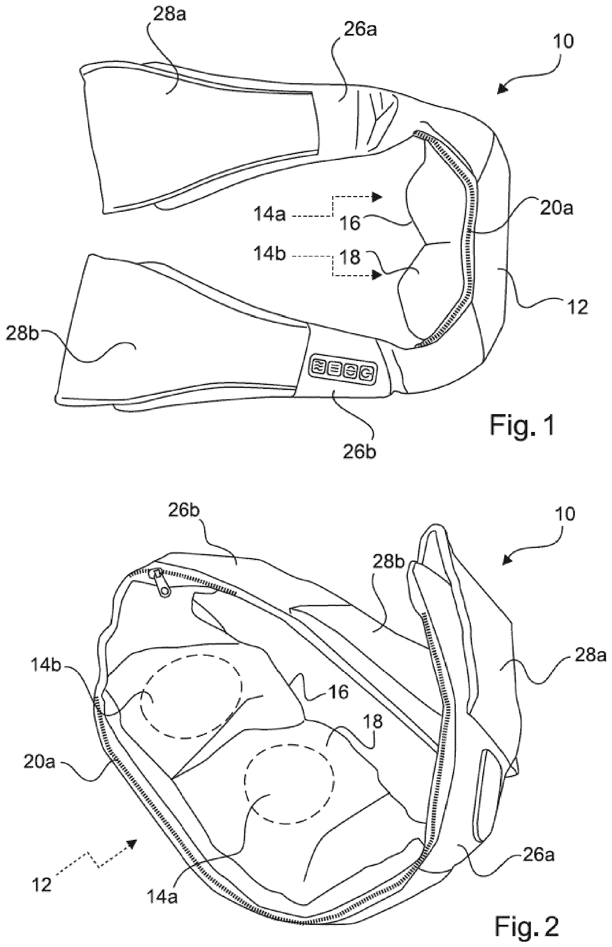

Gemäß Fig. 1 und Fig. 2 umfasst das portable Massagegerät 10 einen Grundkörper 12, in welchem zwei elektrisch antreibbare Massageorgane 14a, 14b angeordnet sind, und ein formveränderliches Kontaktsegment 16.

Die zwei elektrisch antreibbaren Massageorgane 14a, 14b sind dazu eingerichtet, im Betrieb eine Formveränderung des Kontaktsegments 16 zu verursachen. Ferner weisen die zwei elektrisch antreibbaren Massageorgane 14a, 14b jeweils mehrere Massageköpfe auf, welche zum Ausführen einer Knetmassage rotatorisch bewegt werden. Das portable Massagegerät 10 umfasst außerdem mehrere Heizelemente, welche innerhalb des Grundkörpers 12 angeordnet und dazu eingerichtet sind, im Betrieb eine Erwärmung des Kontaktsegments 16 zu verursachen.

Das formveränderliche Kontaktsegment 16 dient zur Auflage auf einen zu massierenden Körperbereich. Das Kontaktsegment 16 weist einen Kontaktbereich 18 auf, welcher dazu eingerichtet ist, den zu massierenden Körperbereich zu berühren. Der Kontaktbereich 18 ist aus einem Textilstoff ausgebildet, wobei der Textilstoff wiederum als Netzstoff ausgebildet ist. Außerdem ist das Kontaktsegment 16 gepolstert.

Das Kontaktsegment 16 ist zerstörungsfrei und reversibel lösbar mittels zweier Reißverschlüsse 20a, 20b mit dem Grundkörper 12 verbunden.

Alternativ oder zusätzlich kann das Kontaktsegment 16 auch mittels eines oder mehrerer Klettverschlüsse und/oder mittels eines oder mehrerer Druckknöpfe mit dem Grundkörper 12 verbunden sein.

Der Grundkörper 12 weist zwei längliche Seitenteile 26a, 26b auf, welche sich jeweils seitlich von dem Kontaktsegment 16 erstrecken. Die länglichen Seitenteile 26a, 26b des Grundkörpers 12 weisen jeweils eine Schlaufe 28a, 28b auf, welche von dem Benutzer gegriffen werden kann.

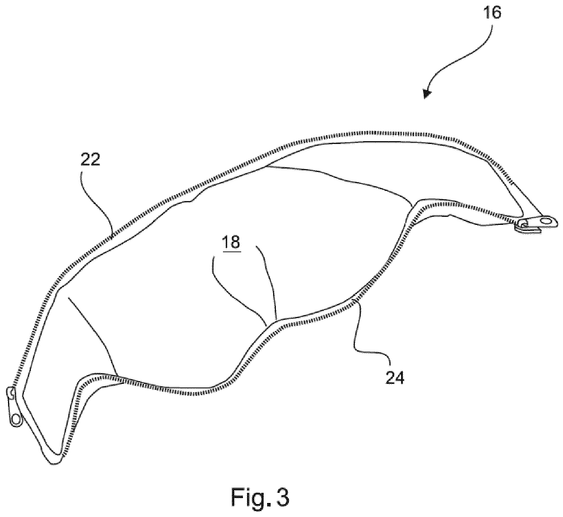

Gemäß Fig. 3 weist das Kontaktsegment 16 eine erste Krampenreihe 22 und eine zweite Krampenreihe 24 auf. Die erste Krampenreihe 22 ist Bestandteil des Reißverschlusses 20a. Die zweite Krampenreihe 24 ist Bestandteil des Reißverschlusses 20b. Die erste Krampenreihe 22 verläuft entlang einer ersten Seitenkante des Kontaktsegments 16 und die zweite Krampenreihe 24 verläuft entlang einer zweiten Seitenkante des Kontaktsegments 16, wobei die erste Seitenkante des Kontaktsegments 16 im Wesentlichen gegenüberliegend zu der zweiten Seitenkante des Kontaktsegments 16 angeordnet ist.

### Bezuqszeichen:

- 10: Massagegerät
- 12: Grundkörper
- 14a, 14b: Massageorgane
- 16: Kontaktsegment
- 18: Kontaktbereich
- 20a, 20b: Reißverschlüsse
- 22, 24: Krampenreihen
- 26a, 26b: längliche Seitenteile
- 28a, 28b: Schlaufen

## Patentansprüche

1. Portables Massagegerät (10), mit
- einem formveränderlichen Kontaktsegment (16) zur Auflage auf einen zu massierenden Körperbereich; und
- einem oder mehreren elektrisch antreibbaren Massageorganen (14a, 14b), welche innerhalb eines Grundkörpers (12) angeordnet und dazu eingerichtet sind, im Betrieb eine Formveränderung des Kontaktsegments (16) zu verursachen;
**dadurch gekennzeichnet, dass** das Kontaktsegment (16) zerstörungsfrei und reversibel lösbar mit dem Grundkörper (12) verbunden ist und wobei das Kontaktsegment (16) mittels eines oder mehrerer Reißverschlüsse (20a, 20b) mit dem Grundkörper (12) verbunden ist.

2. Massagegerät (10) nach Anspruch 1, **dadurch gekennzeichnet, dass** eine oder mehrere Krampenreihen (22, 24) entlang der Seitenkanten des Kontaktsegments (16) verlaufen.

3. Massagegerät (10) nach Anspruch 1 oder 2, **dadurch gekennzeichnet, dass** eine erste Krampenreihe (22) entlang einer ersten Seitenkante des Kontaktsegments (16) und eine zweite Krampenreihe (24) entlang einer zweiten Seitenkante des Kontaktsegments (16) verläuft, wobei die erste Seitenkante des Kontaktsegments (16) im Wesentlichen gegenüberliegend zu der zweiten Seitenkante des Kontaktsegments (16) angeordnet ist.

4. Massagegerät (10) nach einem der vorstehenden Ansprüche, **dadurch gekennzeichnet, dass** das Kontaktsegment (16) mittels eines oder mehrerer Klettverschlüsse mit dem Grundkörper (12) verbunden ist.

5. Massagegerät (10) nach einem der vorstehenden Ansprüche, **dadurch gekennzeichnet, dass** das Kontaktsegment (16) mittels eines oder mehrerer Druckknöpfe mit dem Grundkörper (12) verbunden ist.

6. Massagegerät (10) nach einem der vorstehenden Ansprüche, **dadurch gekennzeichnet, dass** ein Kontaktbereich (18) des Kontaktsegments (16), welcher dazu eingerichtet ist, den zu massierenden Körperbereich zu berühren, zumindest teilweise aus einem Textilstoff ausgebildet ist.

7. Massagegerät (10) nach Anspruch 6, **dadurch gekennzeichnet, dass** der Textilstoff als Netzstoff ausgebildet ist.

8. Massagegerät (10) nach einem der vorstehenden Ansprüche, **dadurch gekennzeichnet, dass** das Kontaktsegment (16) zumindest abschnittsweise gepolstert ist.

9. Massagegerät (10) nach einem der vorstehenden Ansprüche, **dadurch gekennzeichnet, dass** der Grundkörper (12) zwei längliche Seitenteile (26a, 26b) aufweist, welche sich jeweils seitlich von dem Kontaktsegment (16) erstrecken.

10. Massagegerät (10) nach Anspruch 9, **dadurch gekennzeichnet, dass** die länglichen Seitenteile (26a, 26b) des Grundkörpers (12) jeweils eine Schlaufe (28a, 28b) aufweisen.

11. Massagegerät (10) nach einem der vorstehenden Ansprüche, **dadurch gekennzeichnet, dass** das eine oder die mehreren elektrisch antreibbaren Massageorgane (14a, 14b) einen oder mehrere Massageköpfe umfassen.

12. Massagegerät (10) nach einem der vorstehenden Ansprüche, **dadurch gekennzeichnet, dass** das eine oder die mehreren elektrisch antreibbaren Massageorgane (14a, 14b) dazu eingerichtet sind, eine Knetmassage auszuführen.

13. Massagegerät (10) nach einem der vorstehenden Ansprüche, **gekennzeichnet durch** ein oder mehrere Heizelemente, welche innerhalb des Grundkörpers (12) angeordnet und dazu eingerichtet sind, im Betrieb eine Erwärmung des Kontaktsegments (16) zu verursachen.

14. Verwendung eines formveränderlichen Kontaktsegments zum Austauschen des formveränderlichen Kontaktsegments (16) eines Massagegeräts nach einem der Ansprüche 1 bis 13.

## Claims

1. Portable massage appliance (10) having
- a shape-modifiable contact segment (16) for placing onto a body region that is to be massaged; and
- one or more electrically drivable massage elements (14a, 14b), which are arranged inside a main body (12) and are designed to cause a modification of the shape of the contact segment (16) during operation; **characterized in that** the contact segment (16) is connected to the main body (12) so as to be releasable in a non-destructive and reversible manner, and wherein the contact segment (16) is connected to the main body (12) by means of one or more zip fasteners (20a, 20b).

2. Massage appliance (10) according to Claim 1, **characterized in that** one or more rows of teeth (22, 24) run along the side edges of the contact segment (16).

3. Massage appliance (10) according to Claim 1 or 2, **characterized in that** a first row of teeth (22) runs along a first side edge of the contact segment (16) and a second row of teeth (24) runs along a second side edge of the contact segment (16), the first side edge of the contact segment (16) being arranged substantially opposite the second side edge of the contact segment (16) .

4. Massage appliance (10) according to one of the preceding claims, **characterized in that** the contact segment (16) is connected to the main body (12) by means of one or more hook-and-loop fasteners.

5. Massage appliance (10) according to one of the preceding claims, **characterized in that** the contact segment (16) is connected to the main body (12) by means of one or more press studs.

6. Massage appliance (10) according to one of the preceding claims, **characterized in that** a contact region (18) of the contact segment (16), which is designed to touch the body region that is to be massaged, is formed at least partially from a textile fabric.

7. Massage appliance (10) according to Claim 6, **characterized in that** the textile fabric is formed as a net material.

8. Massage appliance (10) according to one of the preceding claims, **characterized in that** the contact segment (16) is padded at least in some parts.

9. Massage appliance (10) according to one of the preceding claims, **characterized in that** the main body (12) has two elongate side parts (26a, 26b), which each extend laterally from the contact segment (16).

10. Massage appliance (10) according to Claim 9, **characterized in that** the elongate side parts (26a, 26b) of the main body (12) each have a loop (28a, 28b).

11. Massage appliance (10) according to one of the preceding claims, **characterized in that** the one or more electrically drivable massage elements (14a, 14b) comprise one or more massage heads.

12. Massage appliance (10) according to one of the preceding claims, **characterized in that** the one or more electrically drivable massage elements (14a, 14b) are designed to perform a kneading massage.

13. Massage appliance (10) according to one of the preceding claims, **characterized by** one or more heating elements, which are arranged inside the main body (12) and are designed to cause heating of the contact segment (16) during operation.

14. Use of a shape-modifiable contact segment for replacing the shape-modifiable contact segment (16) of a massage appliance according to one of Claims 1 to 13.

## Revendications

1. Appareil de massage portable (10), comprenant
- un segment de contact de forme variable (16) destiné à reposer sur une zone du corps à masser ; et
- un ou plusieurs organes de massage entraînables électriquement (14a, 14b), qui sont agencés à l'intérieur d'un corps de base (12) et qui sont conçus pour provoquer un changement de forme du segment de contact (16) pendant le fonctionnement ;
**caractérisé en ce que** le segment de contact (16) est relié de manière non destructive et réversible au corps de base (12) et le segment de contact (16) est relié au corps de base (12) au moyen d'une ou plusieurs fermetures à glissière (20a, 20b).

2. Appareil de massage (10) selon la revendication 1, **caractérisé en ce qu'**une ou plusieurs rangées de crampons (22, 24) s'étendent le long des bords latéraux du segment de contact (16).

3. Appareil de massage (10) selon la revendication 1 ou 2, **caractérisé en ce qu'**une première rangée de crampons (22) s'étend le long d'un premier bord latéral du segment de contact (16) et une deuxième rangée de crampons (24) s'étend le long d'un deuxième bord latéral du segment de contact (16), le premier bord latéral du segment de contact (16) étant agencé essentiellement à l'opposé du deuxième bord latéral du segment de contact (16).

4. Appareil de massage (10) selon l'une quelconque des revendications précédentes, **caractérisé en ce que** le segment de contact (16) est relié au corps de base (12) au moyen d'une ou plusieurs fermetures autoagrippantes.

5. Appareil de massage (10) selon l'une quelconque des revendications précédentes, **caractérisé en ce que** le segment de contact (16) est relié au corps de base (12) au moyen d'un ou plusieurs boutons-poussoirs.

6. Appareil de massage (10) selon l'une quelconque des revendications précédentes, **caractérisé en ce qu'**une zone de contact (18) du segment de contact (16), qui est conçue pour venir en contact avec la zone du corps à masser, est au moins partiellement formée en un matériau textile.

7. Appareil de massage (10) selon la revendication 6, **caractérisé en ce que** le matériau textile est configuré sous la forme d'un matériau à mailles.

8. Appareil de massage (10) selon l'une quelconque des revendications précédentes, **caractérisé en ce que** le segment de contact (16) est rembourré au moins par sections.

9. Appareil de massage (10) selon l'une quelconque des revendications précédentes, **caractérisé en ce que** le corps de base (12) présente deux parties latérales allongées (26a, 26b) qui s'étendent chacune latéralement à partir du segment de contact (16).

10. Appareil de massage (10) selon la revendication 9, **caractérisé en ce que** les parties latérales allongées (26a, 26b) du corps de base (12) présentent chacune un passant (28a, 28b).

11. Appareil de massage (10) selon l'une quelconque des revendications précédentes, **caractérisé en ce que** le ou les organes de massage entraînables électriquement (14a, 14b) comprennent une ou plusieurs têtes de massage.

12. Appareil de massage (10) selon l'une quelconque des revendications précédentes, **caractérisé en ce que** le ou les organes de massage entraînables électriquement (14a, 14b) sont conçus pour effectuer un massage par pétrissage.

13. Appareil de massage (10) selon l'une quelconque des revendications précédentes, **caractérisé par** un ou plusieurs éléments chauffants, qui sont agencés à l'intérieur du corps de base (12) et qui sont conçus pour provoquer un chauffage du segment de contact (16) pendant le fonctionnement.

14. Utilisation d'un segment de contact de forme variable pour le remplacement du segment de contact de forme variable (16) d'un appareil de massage selon l'une quelconque des revendications 1 à 13.
